# EUROPEAN PATENT SPECIFICATION

(11) **EP 4 405 007 B1**
(45) Date of publication and mention of the grant of the patent: **25.11.2026**
(21) Application number: 22789220.5
(22) Date of filing: 22.09.2022
(51) Int. Cl.: A61M 5/315

(54) **ENCODER RING, DOSE RECORDING SYSTEM AND DRUG DELIVERY DEVICE HEREWITH**
CODIERRING, DOSISAUFZEICHNUNGSSYSTEM UND ARZNEIMITTELABGABEVORRICHTUNG DAMIT
ANNEAU DE CODEUR, SYSTÈME D'ENREGISTREMENT DE DOSE ET DISPOSITIF D'ADMINISTRATION DE MÉDICAMENTS ASSOCIÉ

(30) Priority: 24.09.2021 EP 21315185
(43) Date of publication of application: 31.07.2024
(73) Proprietor: Sanofi, 75017 Paris (FR)
(72) Inventor: DRAPER, Paul, Richard, Warwick, CV34 4AB (GB); MORRIS, Anthony, Paul, Warwick, CV34 4AB (GB); STEEL, Samuel, Keir, Warwick, CV34 4AB (GB)
(74) Representative: Keil & Schaafhausen Patentanwälte PartGmbB
(86) International application number: PCT/EP2022/076282
(87) International publication number: WO 2023/046787

(56) References cited:
- EP-B1- 2 620 178
- WO-A1-2021/116388
- CA-A1- 3 073 279
- CN-U- 205 574 971
- US-A1- 2020 360 614
- US-B1- 8 439 922

## Description

The present invention is generally directed to an encoder ring for a dose recording system used in or with a drug delivery device. The present invention further relates to a drug delivery device, which preferably comprises the encoder ring and/or the dose recording system. In more detail, the present invention relates to features of an electronic dose recording system that can be embodied as a non-detachable built-in module or a re-usable clip-on module with a suitably configured pen injector for the purpose of recording doses that are delivered from it.

Pen type drug delivery devices have application where regular injection by persons without formal medical training occurs. This may be increasingly common among patients having diabetes where self-treatment enables such patients to conduct effective management of their disease. In practice, such a drug delivery device allows a user to individually select and dispense a number of user variable doses of a medicament.

There are basically two types of drug delivery devices: resettable devices (i.e., reusable) and non-resettable (i.e., disposable). For example, disposable pen delivery devices are supplied as self-contained devices. Such self-contained devices do not have removable pre-filled cartridges. Rather, the pre-filled cartridges may not be removed and replaced from these devices without destroying the device itself. Consequently, such disposable devices need not have a resettable dose setting mechanism. The present invention is applicable for disposable and reusable devices.

For such devices the functionality of recording doses that are dialled and delivered from the pen may be of value to a wide variety of device users as a memory aid or to support detailed logging of dose history. Thus, drug delivery devices using electronics are becoming increasingly popular in the pharmaceutical industry as well as for users or patients. For example, a drug delivery device is known from WO 2016/131713 A1 comprising an electronically data collection device.

This data collection device comprises a number sleeve rotatable during dose setting and dose dispensing. The number sleeve comprises a castellation at its proximal end. An optical sensor is arranged in the data collection device such that rotation of the castellation wth respect to the sensor may be detected. A similar encoder system for a drug delivery device with an optical sensor is known from WO 2019/101962 A1 and US 2020/360614 A. This encoder system comprises a rotatable dial sleeve with specialy adapted regions divided into reflective areas and non-reflective (absorbent) areas forming together an encoder ring. For a dial sleeve with different reflective areas manufacturing might become difficult while maintaining high accuracy of the geometry.

It is an object of the present disclosure to provide improvements regarding a reliable dose recording system for drug delivery devices which simplifies manufacture and assembly.

This object is solved for example by the subject matter defined in the independent claim, defining the invention.

Advantageous embodiments and refinements are subject to the dependent claims. However, it should be noted that this disclosure may comprise other examples useful for understanding the invention in addition or as an alternative to the ones defined in the appended claims as will become apparent from the following description.

One aspect of the disclosure relates to an encoder ring for a dose recording system of a drug delivery device. The encoder ring may comprise a support ring having a substantially circular configuration with a central axis and comprising a proximal end face and a distal end face, and a series of flag segments rigidly connected to each other via the support ring. Each flag segment has a substantially rectangular outer surface extending in a curved plane. The curved plane in which all flag segments extend is a plane curved in one direction, e.g. a plane defining a vertical circular cylinder surface being coaxially arranged with the central axis of the support ring. In other words, the flag segments may each be a portion of a cylindrical surface, so that they always reflect normal to a sensor located radially outside of the encoder ring. For example, the flag segments may be substantially flush with the proximal end face of the support ring and extend distally beyond the distal end face of the support ring. According to the invention, at least one of the flag segments comprises a retention clip and at least one of the flag segments comprises a positioning pin for fixing and aligning the encoder ring within a dose recording system and/or a drug delivery device.

In an example, one or more, e.g. two non adjacent, flag segments of the encoder ring each comprise a retention clip protruding radially inwards from the distal end of the respective flag segment. Preferably, two flag segments which are located opposite to each other each comprise one retention clip. The flag segment may be elastically deformable to permit snap engagement of a retention clip in a corresponding recess provided in a dose recording system and/or a drug delivery device. Alignment and fixation of the encoder ring may be further improved if one or more, e.g. four, flag segments each comprise a positioning pin protruding distally from the distal end of the respective flag segment. This orientation facilitates bringing the encoder ring in a desired orientation with respect to a dose recording system and/or a drug delivery device when mounting the encoder ring.

Thus, an encoder ring according to the present disclosure may comprise at least one flag segment which comprises a retention clip and at least one flag segment which comprises a positioning pin. This has the advantage of an increased robustness of the dose recording system of a drug delivery device which leads to an increased dose recording accuracy, a lower risk of damages in drop tests of the drug delivery device and a lower risk of unintended disassembly during use of the device.

A retention clip of the encoder ring has the function of axially retaining or fixing the encoder ring on a component part of the drug delivery device, e.g. a dosing sleeve or the like. In addition, the encoder ring is radially held in place by the retention clip. Thus, the retention clip may have two functions of securing the encoder ring on a dosing sleeve or the like. A positioning pin of the encoder ring has the function of radially retaining or fixing the encoder ring on a component part of the drug delivery device, e.g. a dosing sleeve or the like.

The purpose of the flag segments is to allow detection of a rotational position of the encoder ring by means of an, e.g. optical, sensor. Thus, the number and space between the flag segments has an influence on the number of dose increments detactable per revolution of the encoder ring. For example, the flag segments may be equispaced over the circumference and each flag segment may extend over 30° of the circumference.

If an optical sensor is used for rotation encoding, at least the substantially rectangular outer surface, i.e. a portion of a cylindrical surface, of each flag segment may have a surface finish reflecting IR light, especially reflecting NIR light, and/or may be made from a white polymer material containing titanium oxide. If the space between the flag segments is void or made from a less reflective material, an optical sensor may detect rotation of the encoder ring.

The precision of the rotation encoding may be increased when attaching the encoder ring on a rotatable component part with minimum play. In addition to the positioning pins or as an alternative, the flag segments may have a dove tail shape in a cross section perpendicular to the central axis of the encoder ring, i.e. the axis defining the center of the support ring.

According to a further aspect of the present disclosure, a dose recording system for a drug delivery device may comprise a dosing sleeve, at least one optical sensor, a processor, and an encoder ring. For example, the dosing sleeve may be rotatable during a dose seting and/or dose dispensing operation and may comprise a series of flag areas. The processor may be configured to control operation of the at least one optical sensor and to process and/or store signals from the at least one optical sensor. Preferably, the encoder ring is rigidly fixed on the dosing sleeve by means of the at least one retention clip and/or the at least one positioning pin such that the flag segments of the encoder ring are circumferentially interposed between flag areas of the dosing sleeve.

The at least one optical sensor may be positioned radially outside of the encoder ring and the dosing sleeve. In an alternative arrangement, the flag segments and flag areas may be facing radially inwards and the at least one optical sensor may be positioned radially inside of the encoder ring and the dosing sleeve.

If more then one sensor is used, e.g. when using two optical sensors, the optical sensors may be arranged with an angular offset with respect to each other, thereby generating a 4-state gray code pattern during relative rotation of the encoder ring. For example, dose setting and/or dose dispensing can be detected with a 1 international unit (1 IU) resolution using only twelve encoder segments, i.e. six flag segments and six flag areas, while a drug delivery device, like the pen as described in WO2014033195, expels 24 IU per full relative rotation (15° increment per IU) if two optical sensors are provided circumferentially offset by n*30°+15° with n being an integer number (i.e. half of encoder ring segment). Gray code encoding means that only one of the two sensor signals will change per 1 IU (e.g. 15°) rotation of the encoder to avoid ambiguities and race conditions.

In the dose recording system the dosing sleeve may comprise recesses for receiving the at least one retention clip and/or the at least one positioning pin. Further, in an example, the flag areas may be equispaced over the circumference and each flag area may extend over 30° of the circumference.

At least the flag areas of the dosing sleeve may have a surface finish absorbing IR light, especially absorbing NIR light and/or is made from a polymer material containing carbon black. For the purpose of rotation encoding the reflectivity may be opposite to this example, e.g. with a high reflectivity of the flag areas of the dosing sleeve and a lower reflectivity of the flag segments of the encoder ring. In addition or as an alternative, the flag areas of the dosing sleeve may also be positioned further away from the sensors thereby reducing the amount of light reflected.

In the dose recording system, the dosing sleeve may comprise dove tail shaped recesses adjacent to the flag areas for receiving the flag segments of the encoder ring. This permits securing the encoder ring in a precise rotational orientation with respect to the dosing sleeve.

The dose recording system may be an electronic system for use with a drug delivery device suitable for recording doses that are delivered from the drug delivery device. The electronic system may comprise an electrical power supply, e.g. a battery, like a coin cell type battery, a memory for storing data, a processor configured to control operation of the electronic system and coupled to the electrical power supply and to the memory. In addition, the electronic system may comprise at least two optical sensor units, e.g. a first light source with a corresponding first optical sensor and a second light source with a corresponding second optical sensor, which are in communication with the processor. The optical sensors may be suitable for detecting a movement of an encoder of the drug delivery device, especially the flag segments of the encoder ring in conjunction with the flag areas of the dosing sleeve, wherein the movement is indicative of doses that are dialed (i.e. selected) and/or delivered from the drug delivery device. There are several different ways suitable to implement the optical sensor units. For example, the optical sensor unit(s) may comprise a radiation detector comprising an electromagnetic radiation emitter, e.g. an LED, like an IR-LED, e.g. an NIR-LED, and a radiation detector.

In one example, the encoder and the optical sensor units are in an quadrature arrangement, i.e. they are a quarter wave out of phase, which means that if both light sources simultaneously emit light, only one sensor changes state for each unit dispensed. For example, this is achieved by providing two optical sensors circumferentially offset by n*30°+15° with n being an integer number. As the encoder and the sensor units are moved relative to each other, one of the optical sensors which previously received the light now does not receive the emitted light or vice versa. This may be achieved by the encoder selectively reflecting light. For example, the flag segments may reflect light, whereas the flag areas may absorb light. As an alternative, the encoder may selectively block light. In other examples the encoder and the optical sensor units may be in anti-phase arrangement. In a still further alternative, the encoder and the optical sensor units are not in an anti-phase arrangement, such that if both light sources simultaneously emit light, none or only one or all optical sensors detect the light depending on the relative position of the encoder.

The electronic dose recording system may be configured as a re-usable clip-on module for an injection device. As an alternative, the electronic system may be a unit or module integrated (built in) into an injection device. The terms electronic system and (electronic) module are used synonymously in the following for both alternatives. The functionality of recording doses may be of value to a wide variety of device users as a memory aid or to support detailed logging of dose history. It is envisaged that the electronic system, e.g. an electronic module, could be configured to be connectable to a mobile phone, or similar, to enable the dose history to be downloaded from the system on a periodic basis.

The electronic dose recording system may further comprise a communication unit for communicating with another device. Preferably, the electronic dose recording system is configured such that it may be switched from a first state having lower energy consumption into the second state having higher energy consumpton, thereby inducing the communication unit to establish said communication with another device, e.g. a syncronisation or pairing operation. An electronic control unit may issue a command, e.g. a signal, to another unit of the electronic dose recording system such that this unit is switched on or rendered operational. This unit may be the communication unit for communicating with another device, e.g. a wireless communications interface for communicating with another device via a wireless network such as Wi-Fi or Bluetooth, or even an interface for a wired communications link, such as a socket for receiving a Universal Series Bus (USB), mini-USB or micro-USB connector. Preferably, the electronic dose recording system comprises an RF, WiFi and/or Bluetooth unit as the communication unit. The communication unit may be provided as a communication interface between the dose recording system or the drug delivery device and the exterior, such as other electronic devices, e.g. mobile phones, personal computers, laptops and so on. For example, dose data may be transmitted by the communication unit to the external device. The dose data may be used for a dose log or dose history established in the external device.

According to a still further aspect of the present disclosure, the electronic dose recording system further has a sleeping state in which the light sources are not activated (not provided with power from the power source). The electronic dose recording system may further comprise at least one motion sensor suitable for detecting movement of the electronic system. In this example, the processor may be configured to maintain the sleeping state if no movement is detected by the at least one motion sensor and to switch into the first low-power-consumption state or into the at least one further state if a movement is detected by the at least one motion sensor. Generally, a sleeping state or mode may be a mode in which all functionalities of the module are at minimal or virtually zero power consumption but which does not require a system boot up in the event that the electronic system (or the drug delivery device) is taken out of sleeping mode.

For example, such a motion sensor may be used while the electronic dose recording system is in its low-power mode to detect whether the module is stationary. If no movement is detected, the module is assumed to be in storage or idle, with no user interaction occurring, the optical sensors are not polled and the electronic system or module does not wake. If movement is detected, it is assumed that the user may be interacting with the module, the electronic system or module partially wakes into the low-frequency polling mode or into the moderate-frequency polling mode described above. This reduces the amount of power being drawn when the module is sitting stationary, and increases battery life.

If the electronic dose recording system is a re-usable module for releasable attachment to a drug delivery device, the electronic dose recording system or module may comprise an outer cap with a central axis, a chassis which is at least partially retained within the cap and a PCB comprising the memory and the processor. For example, the PCB and the electrical power supply may be retained in the cap and the chassis. Further, the light sources and the optical sensors may be arranged on a circular region about the central axis, with the first light source and the first optical sensor being angularly offset from the second light source and the second optical sensor.

According to a still further aspect of the present disclosure, a drug delivery device for setting and dispensing variable doses of a liquid drug may comprise a cartridge containing a liquid drug and a dose setting and drive mechanism which is configured to perform a dose dialing operation for selecting a dose to be delivered by the drug delivery device and a dose delivery operation for delivering the set dose wherein the dose setting and drive mechanism comprises the dose recording system and/or the encoder ring. For example, the dose recording system may be integrated, e.g. permanently integrated, in a button assembly located at the proximal end of the drug delivery device. The drug delivery device may be a resable device permitting replacement of an empty cartridge. For example, the cartridge may be received in a releasably attached cartridge holder.

In one embodiment, the drug delivery device comprises a dial sleeve, e.g. a number sleeve, or a member axially and/or rotationally locked thereto which is rotatable relative to a housing as the dosing sleeve of the dose setting and drive mechanism, e.g. along a helical path, at least in the dose setting operation. In addition, a dose and/or injection button or a member axially and/or rotationally locked thereto may be axially displaceable relative to the dial sleeve and rotationally constrained to the housing at least in the dose delivery operation. According to one aspect of the disclosure, the encoder ring is fixed to the dosing sleeve during assembly of the drug delivery device. The encoder ring may be, permanently or releasably, clipped on the dosing sleeve.

The present disclosure is applicable for devices which are manually driven, e.g. by a user applying a force to an injection button, for devices which are driven by a spring or the like and for devices which combine these two concepts, i.e. spring assisted devices which still require a user to exert an injection force. The spring-type devices involve springs which are preloaded and springs which are loaded by the user during dose selecting. Some stored-energy devices use a combination of spring preload and additional energy provided by the user, for example during dose setting.

The present disclosure further pertains to a drug delivery device with the electronic system as described above which drug delivery device comprises a cartridge containing a medicament.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide. Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope of the present invention, which is defined by the appended claims.

An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

As further described in ISO 11608-1:2014(E), a multi-dose container system may involve a needle-based injection device with a replaceable container. In such a system, each container holds multiple doses, the size of which may be fixed or variable (pre-set by the user). Another multi-dose container system may involve a needle-based injection device with an integrated non-replaceable container. In such a system, each container holds multiple doses, the size of which may be fixed or variable (pre-set by the user).

As further described in ISO 11608-1:2014(E), a single-dose container system may involve a needle-based injection device with a replaceable container. In one example for such a system, each container holds a single dose, whereby the entire deliverable volume is expelled (full evacuation). In a further example, each container holds a single dose, whereby a portion of the deliverable volume is expelled (partial evacuation). As also described in ISO 11608-1:2014(E), a single-dose container system may involve a needle-based injection device with an integrated non-replaceable container. In one example for such a system, each container holds a single dose, whereby the entire deliverable volume is expelled (full evacuation). In a further example, each container holds a single dose, whereby a portion of the deliverable volume is expelled (partial evacuation).

The terms "axial", "radial", or "circumferential" as used herein may be used with respect to a main longitudinal axis of the device, the cartridge, the housing or the cartridge holder, e.g. the axis which extends through the proximal and distal ends of the cartridge, the cartridge holder or the drug delivery device.

Non-limiting, exemplary embodiments of the disclosure will now be described with reference to the accompanying drawings, in which:
- Figure 1: shows an embodiment of a drug delivery device;
- Figure 2: shows an encoder ring and a dosing sleeve of a first embodiment of a dose recording system prior to assembly;
- Figure 3: shows the encoder ring and the dosing sleeve of Figure 2 after assembly;
- Figure 4: shows in a sectional view the encoder ring and the dosing sleeve of Figure 2; and
- Figure 5: shows in a further sectional view the encoder ring and the dosing sleeve of Figure 2.

In the figures, identical elements, identically acting elements or elements of the same kind may be provided with the same reference numerals.

In the following, some embodiments will be described with reference to an insulin injection device. The present disclosure is however not limited to such application and may equally well be deployed with injection devices that are configured to eject other medicaments or drug delivery devices in general, preferably pen-type devices and/or injection devices.

Embodiments are provided in relation to injection devices, in particular to variable dose injection devices, which record and/or track data on doses delivered thereby. These data may include the size of the selected dose and/or the size of the actually delivered dose, the time and date of administration, the duration of the administration and the like. Features described herein include the arrangement of sensing elements and power management techniques (e.g. to facilitate small batteries and/or to enable efficient power usage).

Certain embodiments in this document are illustrated with respect to injection device as described in WO2014033195 where an injection button and grip (dose setting member or dose setter) are combined. The injection button may provide the user interface member for initiating and/or performing a dose delivery operation of the drug delivery device. The grip or knob may provide the user interface member for initiating and/or performing a dose setting operation. Both devices are of the dial extension type, i.e. their length increases during dose setting. Other injection devices with the same kinematical behaviour of the dial extension and button during dose setting and dose expelling operational mode are known as, for example, the Kwikpen^{®} device marketed by Eli Lilly and the Novopen^{®} 4 device marketed by Novo Nordisk. An application of the general principles to these devices therefore appears straightforward and further explanations will be omitted. However, the general principles of the present disclosure are not limited to that kinematical behaviour. Certain other embodiments may be conceived for application to an injection device as described in WO2004078239 where there are separate injection button and grip components / dose setting members. Thus, there may be two separate user interface members, one for the dose setting operation and one for the dose delivery operation.

"Distal" is used herein to specify directions, ends or surfaces which are arranged or are to be arranged to face or point towards a dispensing end of the drug delivery device or components thereof and/or point away from, are to be arranged to face away from or face away from the proximal end. On the other hand, "proximal" is used to specify directions, ends or surfaces which are arranged or are to be arranged to face away from or point away from the dispensing end and/or from the distal end of the drug delivery device or components thereof. The distal end may be the end closest to the dispensing and/or furthest away from the proximal end and the proximal end may be the end furthest away from the dispensing end. A proximal surface may face away from the distal end and/or towards the proximal end. A distal surface may face towards the distal end and/or away from the proximal end. The dispensing end may be the needle end where a needle unit is or is to be mounted to the device, for example.

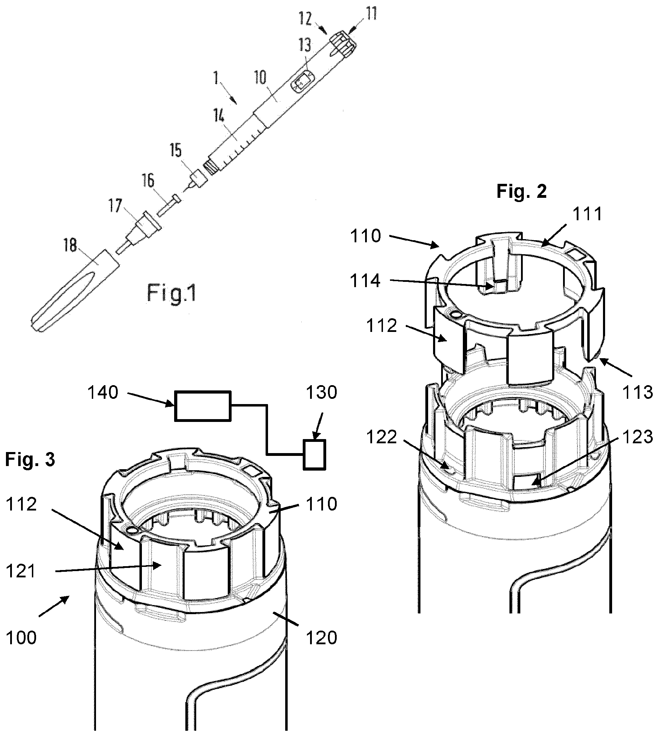

Figure 1 is an exploded view of a medicament delivery device or drug delivery device. In this example, the medicament delivery device is an injection device 1, e.g. a pen-type injector, such as the injection pen described in WO2014033195.

The injection device 1 of Figure 1 is an injection pen that comprises a housing 10 and contains a container 14, e.g. an insulin container, or a receptacle for such a container. The container may contain a drug. A needle 15 can be affixed to the container or the receptacle. The container may be a cartridge and the receptacle may be a cartridge holder. The needle is protected by an inner needle cap 16 and either an outer needle cap 17 or another cap 18. An insulin dose to be ejected from injection device 1 can be set, programmed, or 'dialled in' by turning a dosage knob 12, and a currently programmed or set dose is then displayed via dosage window 13, for instance in multiples of units. The indicia displayed in the window may be provided on a number sleeve or dial sleeve. For example, where the injection device 1 is configured to administer human insulin, the dosage may be displayed in so-called International Units (IU), wherein one IU is the biological equivalent of about 45.5 micrograms of pure crystalline insulin (1/22 mg). Other units may be employed in injection devices for delivering analogue insulin or other medicaments. It should be noted that the selected dose may equally well be displayed differently than as shown in the dosage window 13 in Figure 1.

The dosage window 13 may be in the form of an aperture in the housing 10, which permits a user to view a limited portion of a dial sleeve (e.g. dosing sleeve 120 of Fihures 2 and 3) that is configured to move when the dosage knob 12 is turned, to provide a visual indication of a currently set dose. The dosage knob 12 is rotated on a helical path with respect to the housing 10 when setting a dose. In this example, the dosage knob 12 includes one or more formations to facilitate attachment of a data collection device.

The injection device 1 may be configured so that turning the dosage knob 12 causes a mechanical click sound to provide acoustic feedback to a user. In this embodiment, the dosage knob or dose button 12 also acts as an injection button 11. When needle 15 is stuck into a skin portion of a patient, and then dosage knob 12 / injection button 11 is pushed in an axial direction, the insulin dose displayed in display window 13 will be ejected from injection device 1. When the needle 15 of injection device 1 remains for a certain time in the skin portion after the dosage knob 12 is pushed, the dose is injected into the patient's body. Ejection of the insulin dose may also cause a mechanical click sound, which may be different from the sounds produced when rotating the dosage knob 12 during dialing of the dose.

In this embodiment, during delivery of the insulin dose, the dosage knob 12 is returned to its initial position in an axial movement, without rotation, while the dial sleeve is rotated to return to its initial position, e.g. to display a dose of zero units. As noted already, the disclosure is not restricted to insulin but should encompass all drugs in the drug container 14, especially liquid drugs or drug formulations.

Injection device 1 may be used for several injection processes until either the insulin container 14 is empty or the expiration date of the medicament in the injection device 1 (e.g. 28 days after the first use) is reached. In the case of a resuable device, it is possible to replace the insulin container.

Furthermore, before using injection device 1 for the first time, it may be necessary to perform a so-called "prime shot" to remove air from insulin container 14 and needle 15, for instance by selecting two units of insulin and pressing dosage knob 12 while holding injection device 1 with the needle 15 upwards. For simplicity of presentation, in the following, it will be assumed that the selected amounts substantially correspond to the injected doses, so that, for instance the amount of medicament selected from the injection device 1 is equal to the dose received by the user.

As explained above, the dosage knob 12 also functions as an injection button 11 so that the same component is used for dialling/setting the dose and dispensing/delivering the dose.

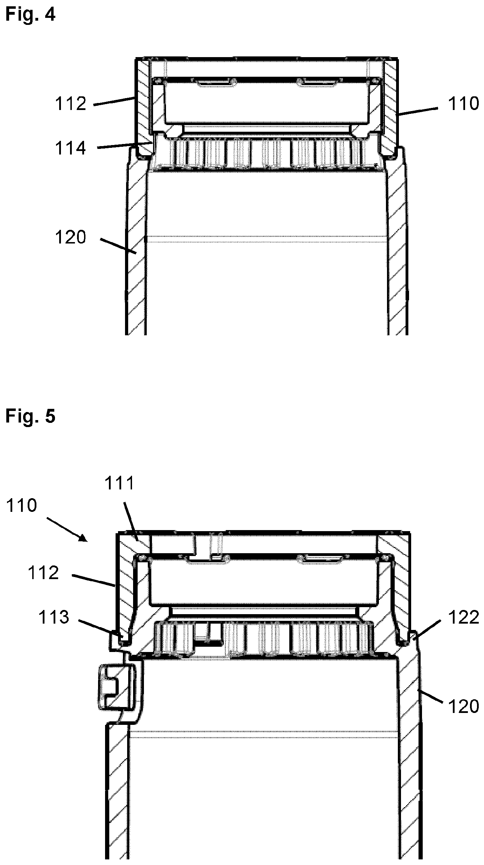

In the following, an electronic dose recording system 100 according to the invention will be described with respect to Figures 2 to 5. In an exemplary embodiment, the electronic dose recording system 100 may consitute a re-usable module which may be releasably attached to the injection device 1, specifically to the button 11 and the dosage knob 12. The central longitudinal axis of the injection device 1 is identical with the central axis of the electronic module 100. The general working principle of the module 100, its design and interaction with an injection device 1 may be similar as disclosed in unpublished EP 20 315 451.3 and PCT/EP2020/085728 to which reference is made.

The dose recording system comprises an encoder ring 110 which may be attached to a dosing sleeve 120, at least one optical sensor 130, e.g. two optical sensors 130, and a processor which may be part of a PCB unit 140.

The depicted encoder ring 110 consists of a substantially circular support ring 111 with a proximal end face and a distal end face, and a series of flag segments 112, e.g. six flag segments 112 as depicted in Figure 2. The flag segments 112 are formed as a portion of a cylinder surface having a substantially rectangular outline or shape, at least at the outer surface, and extend in a curved plane, like in a cylindrical plane as in the depicted embodiment. The flag segments 112 are rigidly connected to each other via the support ring 111. In the depicted example, the proximal end of each flag segment 112 is substantially in the plane defined by the proximal end face of the support ring 111, whereas the flag segments 112 distally protrude over the distal end face of the support ring 111. Each flag segment 112 may have a dove tail shape in cross section.

In the depicted example, four flag segments 112 comprise a positioning pin 113 extending distally from the distal end face of the respective flag segment 112. The number and orientation of the positioning pins 113 may depart from the depicted example. Further, the exemplary embodiment comprises retention clips 114 on two opposite located flag segments 112. The retention clips are formed as a radially inwards extending protrusion near the distal end of the respective flag segment 112.

The encoder ring 110 may be a unitary component part formed by injection moulding. For example, a gate point may be on the top of the encoder ring 110, located in a wider area that does not interface with the flag geometries. In addition, a cavity for providing an ID may be placed on the top of the encoder ring 110, on the opposite side of the gate point place.

The encoder ring 110 must provide radial clearance to other components of the drug delivery device when assembled, e.g. to a chassis. A too large flag segement wall thickness or a too large/oval outer diameter or a small/oval inner diameter could lead to friction between such a chassis and the encoder ring 110 during dispensing. This friction is typically undesired as torsional drag induces increased dispense force. In the worst case, the device stalls during dispense. Additionally, a too large flag segment wall thickness or a small/oval inner diameter could lead to interference between a chassis and the encoder ring 110, that provokes the module returning to the fully proximal position. This may result in switch contacts not closing such that a microcontroller could go to sleep and no switch transition occurs to ever wake up again.

The dosing sleeve 120 is a tubular element with a series of flag areas 121 at its proximal end. In the depicted embodiment, six flag areas 121 are provided with recesses therebetween for receiving the flag segments 112 of encoder ring 110 when attached to the dosing sleeve as shown in Figure 3. Further, the dosing sleeve 120 comprises distally extending recesses 122 for receiving a respective positioning pin 113 and radially extending recesses 123 for receiving a respective retention clip 114 of the encoder ring 110.

For example, the dosing sleeve 120 may be a multi component part comprising the portion with the flag areas 121 and a separate number sleeve (not shown). Once assembled, no relative movement is allowed between these component parts. The parts may be made as separate components to enable both moulding and assembly. Also, whereas the number sleeve may be white to give contrast for black dose numbers provided on the number sleeve, the colour of the portion comprising the flag areas can be chosen to be non-reflective to the light from the sensors ,e.g. grey/black, suit the aesthetics or perhaps to distinguish the drug type.

The encoder ring 110 or at least its flag segments 112 and the dosing sleeve 120 or at least its flag areas 121 are made from different materials and/or are provided with a different surface finish such that the reflectivity of the flag segments 112 differs from the reflectivity of the flag areas 121. For example, the flag segments 112 may have a high reflectivity for IR light whereas the flag areas 121 have a significantly lesser reflectivity for IR light and/or absorb IR light. This different reflectivity may be detected by the optical sensors 130.

The encoder flag segments 112 are clipped to the dosing sleeve 120 (dial sleeve) to ensure a firm fixation of the encoder ring component 110. The contact between the respective flag walls of the encoder ring 110 and the dosing sleeve 120 must ensure a clear contrast transition (e.g. black and white) to allow an accurate dose increment detection of the optical (photo) sensors 130. The retention clips 114 ensure the correct fixation of the encoder ring 110 to the dosing sleeve 120. In the case that the encoder ring 110 detaches from the dosing sleeve 120 or if the pins aren't there, the flags may deflect outwards radially such that the clearance with further components, e.g. a chassis, is reduced leading to friction during dispensing, and in the worst case, leading to jammed mechanisms. On the other hand, rotational play between the encoder ring 110 and the dosing sleeve 120 during dosing could give one fewer or one extra encoder flag transitions to the optical sensors 130, leading to unit dose record errors. The positioning pins 113 ensure the correct position (both rotationally and radially) of the encoder ring edges, when attached to the dosing sleeve 120.

The surface finish of the encoder ring 110 flag geometries 112 reflects IR light. An insufficient surface finish might lead to the sensors 130 to miss counting units. The fully attached encoder ring 110 (Figure 3) provides in combination with the dosing sleeve 120 a black and white surfaces (flag segments 112 and flag areas 121), working as contrast surfaces to be detected by sensors 130.

To provide a dose recording functionality, the dial sleeve (dosing sleeve) of a drug delivery device may be modified to hold the clip-on encoder ring component 110 on its distal end. While the dosing sleeve 120 may be moulded with a dark grey material, the encoder ring 110 may be produced from a white opaque material. Thereby, a regular pattern of light and dark angular surface segments is created at the circumference of the distal dosing sleeve end. Each angular segment spans 30° (6 light and 6 dark segments in total) in the depicted example. In an example, the segments 121 are positioned on a smaller diameter than the flags 112, therefore further away from the sensor for further reducing the light reflected.

Dose recording is realized by detecting the relative rotation between the dosing sleeve 120, i.e. its encoder ring portion, and a stationary module which occurs during injection. For this purpose, the electronics integrated in the module incorporate two photo reflector units (i.e. optical sensors 130) arranged radially around the encoder ring 110. The sensors 130 are used to detect and distinguish the light and dark encoder ring surfaces (flag segments 112 and flag areas 121). The sensors 130 are arranged with an angular offset of n*30°+15° (i.e. out of phase by half of one encoder ring segment). Thereby, a 4-State Gray Code pattern is generated during relative rotation and injections can be detected with a 1 IU resolution using only six encoder flag segments 112 with six flag areas 121 while, e.g. the pen as described in WO2014033195, expels 24 IU per full relative rotation (15° increment per IU).

In an example, the photo reflectors incorporate an LED light emitter operating in the near IR band (around 900nm wavelength) and corresponding photo transistors to detect the reflected light from the encoder ring surface. While the light encoder flag segments 112 have a high IR reflection, the dark segments of the flag areas 121 have a high absorption and show low reflection.

Before an injection, the electronics and the sensor system may be activated, e.g. by an axial electrical switch embedded in the module which closes on axial movement of the module toward the pen body and/or the dosing sleeve 120 and before the relative rotation starts during an injection. During an injection, the sensors 130 are driven by pulses from a microcontroller unit (MCU) embedded in the electronics. The photodetectors 130 generate analogue signals which are sampled by the MCU and evaluated within the MCU. If the optical sensors 130 are sampled at a sufficiently high frequency (e.g. 4kHz), the injection speed can be determined in the MCU to detect and flag high speed injections.

### Reference Numerals

- 1: device
- 10: housing
- 11: injection button
- 12: dosage knob
- 13: dosage window
- 14: container/container receptacle
- 15: needle
- 16: inner needle cap
- 17: outer needle cap
- 18: cap

- 100: electrical dose recording system

- 110: encoder ring
- 111: support ring
- 112: flag segment
- 113: positioning pin
- 114: retention clip

- 120: dosing sleeve
- 121: flag area
- 122: recess
- 123: recess

- 130: optical sensor

- 140: PCB unit (processor)

## Claims

1. An encoder ring for a dose recording system of a drug delivery device, the encoder ring comprising:
- a support ring (111) having a substantially circular configuration and comprising a proximal end face and a distal end face, and
- a series of flag segments (112) rigidly connected to each other via the support ring (111), with each flag segment (112) having a substantially rectangular outer surface extending in a curved plane defining a vertical circular cylinder surface being coaxially arranged with the central axis of the support ring (111),
**characterized in that** at least one of the flag segments (112) comprises a retention clip (114) and at least one of the flag segments (112) comprises a positioning pin (113).

2. The encoder ring according to claim 1, wherein two non adjacent flag segments (112) each comprise a retention clip (114) protruding radially inwards from the distal end of the respective flag segment (112).

3. The encoder ring according to any one of the preceding claims, wherein four flag segments (112) each comprise a positioning pin (113) protruding distally from the distal end of the respective flag segment (112).

4. The encoder ring according to any one of the preceding claims, wherein the flag segments (112) are equispaced over the circumference and each flag segment (112) extends over 30° of the circumference.

5. The encoder ring according to any one of the preceding claims, wherein at least the substantially rectangular outer surface of each flag segment (112) has a surface reflecting IR light, especially reflecting NIR light, and/or is made from a white polymer material containing titanium oxide.

6. The encoder ring according to any one of the preceding claims, wherein the flag segments (112) have a dove tail shape in a cross section perpendicular to the central axis of the encoder ring (110).

7. A dose recording system for a drug delivery device comprising:
- a dosing sleeve (120) rotatable during a dose seting and/or dose dispensing operation and comprising a series of flag areas (121),
- at least one optical sensor (130),
- a processor (140) configured to control operation of the at least one optical sensor (130) and to process and/or store signals from the at least one optical sensor (130), and
- an encoder ring (110) according to any one of the preceding claims,
wherein the encoder ring (110) is rigidly fixed on the dosing sleeve (120) by means of the at least one retention clip (114) and/or the at least one positioning pin (113) such that the flag segments (112) of the encoder ring (110) are circumferentially interposed between flag areas (121) of the dosing sleeve (120).

8. The dose recording system according to claims 4 and 7, wherein two optical sensors (130) are provided circumferentially offset by n*30°+15° with n being an integer number.

9. The dose recording system according to any on of claims 7 or 8, wherein the dosing sleeve (120) comprises recesses (122; 123) for receiving the at least one retention clip (114) and/or the at least one positioning pin (113).

10. The dose recording system according to any on of claims 7 to 9, wherein the flag areas (121) are equispaced over the circumference and each flag area (121) extends over 30° of the circumference.

11. The dose recording system according to any on of claims 7 to 10, wherein at least the flag areas (121) have a surface finish absorbing IR light, especially absorbing NIR light and/or is made from a polymer material containing carbon black.

12. The dose recording system according to any on of claims 7 to 11, wherein the dosing sleeve (120) comprises dove tail shaped recesses adjacent the flag areas (121) for receiving the flag segments (112) of the encoder ring (110).

13. A drug delivery device for setting and dispensing variable doses of a liquid drug, the device comprising a cartridge containing a liquid drug and a dose setting and drive mechanism which is configured to perform a dose dialing operation for selecting a dose to be delivered by the drug delivery device and a dose delivery operation for delivering the set dose, the dose setting and drive mechanism comprising the dose recording system according to any one of claims 7 to 12.

14. The drug delivery device according to claim 13, wherein the dose recording system is integrated in a button assembly located at the proximal end of the drug delivery device.

15. The drug delivery device according to claim 13 or 14, wherein the cartridge is received in a releasably attached cartridge holder.

## Patentansprüche

1. Encoderring für ein Dosisaufzeichnungssystem einer Medikamenten-Verabreichungsvorrichtung, wobei der Encoderring Folgendes umfasst:
- einen Tragring (111), der eine im Wesentlichen kreisförmige Konfiguration aufweist und eine proximale Endfläche und eine distale Endfläche umfasst, und
- eine Reihe von Fahnensegmenten (112), die über den Tragring (111) starr miteinander verbunden sind, wobei jedes Fahnensegment (112) eine im Wesentlichen rechteckige Außenoberfläche aufweist, die sich in einer gekrümmten Fläche erstreckt, die eine vertikale kreisförmige Zylinderoberfläche definiert, die koaxial zu der Mittelachse des Tragrings (111) angeordnet ist,
**dadurch gekennzeichnet, dass** mindestens eines der Fahnensegmente (112) einen Halteclip (114) umfasst und mindestens eines der Fahnensegmente (112) einen Positionierstift (113) umfasst.

2. Encoderring nach Anspruch 1, wobei zwei nicht benachbarte Fahnensegmente (112) jeweils einen Halteclip (114) umfassen, der von dem distalen Ende des jeweiligen Fahnensegments (112) radial nach innen vorsteht.

3. Encoderring nach einem der vorhergehenden Ansprüche, wobei vier Fahnensegmente (112) jeweils einen Positionierstift (113) umfassen, der von dem distalen Ende des jeweiligen Fahnensegments (112) distal vorsteht.

4. Encoderring nach einem der vorhergehenden Ansprüche, wobei die Fahnensegmente (112) über den Umfang äquidistant sind und sich jedes Fahnensegment (112) über 30° des Umfangs erstreckt.

5. Encoderring nach einem der vorhergehenden Ansprüche, wobei mindestens die im Wesentlichen rechteckige Außenoberfläche jedes Fahnensegments (112) eine Oberfläche aufweist, die IR-Licht reflektiert, insbesondere NIR-Licht reflektiert, und/oder aus einem weißen, Titanoxid enthaltenden Polymermaterial hergestellt ist.

6. Encoderring nach einem der vorhergehenden Ansprüche, wobei die Fahnensegmente (112) eine Schwalbenschwanzform in einem zu der Mittelachse des Encoderrings (110) senkrechten Querschnitt aufweisen.

7. Dosisaufzeichnungssystem für eine Medikamenten-Verabreichungsvorrichtung, umfassend:
- eine Dosierhülse (120), die während eines Dosiseinstell- und/oder Dosisabgabevorgangs drehbar ist und eine Reihe von Fahnenbereichen (121) umfasst,
- mindestens einen optischen Sensor (130),
- einen Prozessor (140), der dazu ausgelegt ist, den Betrieb des mindestens einen optischen Sensors (130) zu steuern und Signale von dem mindestens einen optischen Sensor (130) zu verarbeiten und/oder zu speichern, und
- einen Encoderring (110) nach einem der vorhergehenden Ansprüche,
wobei der Encoderring (110) mittels des mindestens einen Halteclips (114) und/oder des mindestens einen Positionierstifts (113) an der Dosierhülse (120) starr befestigt ist, so dass die Fahnensegmente (112) des Encoderrings (110) in Umfangsrichtung zwischen Fahnenbereichen (121) der Dosierhülse (120) angeordnet sind.

8. Dosisaufzeichnungssystem nach den Ansprüchen 4 und 7, wobei zwei optische Sensoren (130) in Umfangsrichtung um n*30°+15° versetzt bereitgestellt sind, wobei n eine ganze Zahl ist.

9. Dosisaufzeichnungssystem nach Anspruch 7 oder 8, wobei die Dosierhülse (120) Aussparungen (122; 123) zum Aufnehmen des mindestens einen Halteclips (114) und/oder des mindestens einen Positionierstifts (113) umfasst.

10. Dosisaufzeichnungssystem nach einem der Ansprüche 7 bis 9, wobei die Fahnenbereiche (121) über den Umfang äquidistant sind und sich jeder Fahnenbereich (121) über 30° des Umfangs erstreckt.

11. Dosisaufzeichnungssystem nach einem der Ansprüche 7 bis 10, wobei mindestens die Fahnenbereiche (121) eine Oberflächenbeschaffenheit aufweisen, aufgrund derer sie IR-Licht absorbieren, insbesondere NIR-Licht absorbieren, und/oder aus einem Ruß enthaltenden Polymermaterial hergestellt sind.

12. Dosisaufzeichnungssystem nach einem der Ansprüche 7 bis 11, wobei die Dosierhülse (120) schwalbenschwanzförmige Aussparungen, die zu den Fahnenbereichen (121) benachbart sind, zum Aufnehmen der Fahnensegmente (112) des Encoderrings (110) umfasst.

13. Medikamenten-Verabreichungsvorrichtung zum Einstellen und Abgeben veränderbarer Dosen eines flüssigen Medikaments, wobei die Vorrichtung eine Kartusche, die ein flüssiges Medikament enthält, und einen Dosiseinstell- und Antriebsmechanismus umfasst, der dazu ausgelegt ist, einen Dosiswählvorgang zum Auswählen einer durch die Medikamenten-Verabreichungsvorrichtung zu verabreichenden Dosis und einen Dosisverabreichungsvorgang zum Verabreichen der eingestellten Dosis durchzuführen, wobei der Dosiseinstell- und Antriebsmechanismus das Dosisaufzeichnungssystem nach einem der Ansprüche 7 bis 12 umfasst.

14. Medikamenten-Verabreichungsvorrichtung nach Anspruch 13, wobei das Dosisaufzeichnungssystem in eine an dem proximalen Ende der Medikamenten-Verabreichungsvorrichtung befindliche Knopfbaugruppe integriert ist.

15. Medikamenten-Verabreichungsvorrichtung nach Anspruch 13 oder 14, wobei die Kartusche in einem lösbar angebrachten Kartuschenhalter aufgenommen ist.

## Revendications

1. Anneau de codage pour un système d'enregistrement de dose d'un dispositif d'administration de médicament, l'anneau de codage comprenant :
- un anneau de support (111) ayant une configuration sensiblement circulaire et comprenant une face d'extrémité proximale et une face d'extrémité distale, et
- une série de palettes (112) reliées rigidement entre elles par le biais de l'anneau de support (111), chaque palette (112) présentant une surface externe sensiblement rectangulaire s'étendant dans un plan incurvé définissant une surface cylindrique circulaire verticale qui est agencée coaxialement avec l'axe central de l'anneau de support (111),
**caractérisé en ce qu'**au moins une des palettes (112) comprend une agrafe de retenue (114) et au moins une des palettes (112) comprend une goupille de positionnement (113).

2. Anneau de codage selon la revendication 1, dans lequel deux palettes non adjacentes (112) comprennent chacune une agrafe de retenue (114) faisant saillie radialement vers l'intérieur à partir de l'extrémité distale de la palette (112) respective.

3. Anneau de codage selon l'une quelconque des revendications précédentes, dans lequel quatre palettes (112) comprennent chacune une goupille de positionnement (113) faisant saillie distalement à partir de l'extrémité distale de la palette (112) respective.

4. Anneau de codage selon l'une quelconque des revendications précédentes, dans lequel les palettes (112) sont réparties à intervalles réguliers sur la circonférence et chaque palette (112) s'étend sur 30° de la circonférence.

5. Anneau de codage selon l'une quelconque des revendications précédentes, dans lequel au moins la surface externe sensiblement rectangulaire de chaque palette (112) a une surface réfléchissant la lumière IR, en particulier réfléchissant la lumière NIR, et/ou est fabriquée à partir d'un matériau polymère blanc contenant de l'oxyde de titane.

6. Anneau de codage selon l'une quelconque des revendications précédentes, dans lequel les palettes (112) ont une forme de queue d'aronde dans une coupe transversale perpendiculaire à l'axe central de l'anneau de codage (110).

7. Système d'enregistrement de dose pour un dispositif d'administration de médicament comprenant :
- un manchon de dosage (120) pouvant tourner lors d'une opération de réglage de dose et/ou de distribution de dose et comprenant une série de zones de palettes (121),
- au moins un capteur optique (130),
- un processeur (140) configuré pour commander le fonctionnement de l'au moins un capteur optique (130) et pour traiter et/ou stocker des signaux provenant de l'au moins un capteur optique (130), et
- un anneau de codage (110) selon l'une quelconque des revendications précédentes,
dans lequel l'anneau de codage (110) est fixé à demeure sur le manchon de dosage (120) au moyen de l'au moins une agrafe de retenue (114) et/ou de l'au moins une goupille de positionnement (113) de telle sorte que les palettes (112) de l'anneau de codage (110) soient intercalées circonférentiellement entre des zones de palettes (121) du manchon de dosage (120).

8. Système d'enregistrement de dose selon les revendications 4 et 7, dans lequel deux capteurs optiques (130) sont fournis décalés circonférentiellement de n*30° + 15°, n étant un nombre entier.

9. Système d'enregistrement de dose selon l'une quelconque des revendications 7 ou 8, dans lequel le manchon de dosage (120) comprend des évidements (122 ; 123) pour recevoir l'au moins une agrafe de retenue (114) et/ou l'au moins une goupille de positionnement (113).

10. Système d'enregistrement de dose selon l'une quelconque des revendications 7 à 9, dans lequel les zones de palettes (121) sont réparties à intervalles réguliers sur la circonférence et chaque zone de palette (121) s'étend sur 30° de la circonférence.

11. Système d'enregistrement de dose selon l'une quelconque des revendications 7 à 10, dans lequel au moins les zones de palettes (121) ont une finition de surface absorbant la lumière IR, en particulier absorbant la lumière NIR et/ou sont fabriquées à partir d'un matériau polymère contenant du noir de carbone.

12. Système d'enregistrement de dose selon l'une quelconque des revendications 7 à 11, dans lequel le manchon de dosage (120) comprend des évidements en forme de queue d'aronde adjacents aux zones de palettes (121) pour recevoir les palettes (112) de l'anneau de codage (110).

13. Dispositif d'administration de médicament permettant de régler et de distribuer des doses variables d'un médicament liquide, le dispositif comprenant une cartouche contenant un médicament liquide et un mécanisme de réglage et d'entraînement de dose qui est configuré pour effectuer une opération de sélection de dose permettant de sélectionner une dose devant être administrée par le dispositif d'administration de médicament et une opération d'administration de dose permettant d'administrer la dose réglée, le mécanisme de réglage et d'entraînement de dose comprenant le système d'enregistrement de dose selon l'une quelconque des revendications 7 à 12.

14. Dispositif d'administration de médicament selon la revendication 13, dans lequel le système d'enregistrement de dose est intégré dans un ensemble bouton situé à l'extrémité proximale du dispositif d'administration de médicament.

15. Dispositif d'administration de médicament selon la revendication 13 ou 14, dans lequel la cartouche est reçue dans un support de cartouche fixé de façon amovible.
